# EUROPÄISCHE PATENTSCHRIFT

(11) **EP 2 279 157 B1**
(45) Veröffentlichungstag und Bekanntmachung des Hinweises auf die Patenterteilung: **25.01.2012**
(21) Anmeldenummer: 09723621.0
(22) Anmeldetag: 17.03.2009
(51) Int. Cl.: C07C 7/13, C07C 53/06, C07C 53/10, F17C 11/00, B01D 53/04, C10L 3/06, B01J 20/22

(54) **VERWENDUNG VON FORMIAT-BASIERTEN PORÖSEN METALLORGANISCHEN GERÜSTMATERIALIEN ZUR METHANSPEICHERUNG**
USE OF FORMIATE-BASED POROUS ORGANOMETALLIC FRAMEWORK MATERIALS FOR STORING METHANE
UTILISATION DE MATÉRIAUX STRUCTURELS ORGANOMÉTALLIQUES POREUX À BASE DE FORMIATE POUR LE STOCKAGE DE MÉTHANE

(30) Priorität: 17.03.2008 EP 08152821
(43) Veröffentlichungstag der Anmeldung: 02.02.2011
(73) Patentinhaber: BASF SE, 67056 Ludwigshafen (DE)
(72) Erfinder: LEUNG, Emi, 68161 Mannheim (DE); MÜLLER, Ulrich, 67435 Neustadt (DE); COX, Gerhard, 67098 Bad Dürkheim (DE); HÖFFKEN, Hans, Wolfgang, 67069 Ludwigshafen (DE)
(86) Internationale Anmeldenummer: PCT/EP2009/053130
(87) Internationale Veröffentlichungsnummer: WO 2009/115513

(56) Entgegenhaltungen:
- EP-A- 1 674 555
- WO-A-2007/118841
- DE-B- 1 149 674
- DE-C- 263 865
- US-A- 5 264 623
- US-A- 5 998 647
- ROOD, J.A. ET AL.: "Synthesis, structural characterization, gas sorption and guest-exchange studies of the lightweight, porous metal-organic framework alpha-[Mg3(O2CH)6]" INORGANIC CHEMISTRY, Bd. 45, Nr. 14, 2006, Seiten 5521-5528, XP002539785

## Beschreibung

Die vorliegende Erfindung betrifft die Verwendung eines porösen metallorganischen Gerüstmaterials zur Speicherung oder Abtrennung von Methan, entsprechende Verfahren, poröse metallorganische Gerüstmaterialien sowie deren Herstellung.

Materialien zur Speicherung oder Trennung von Gasen sind im Stand der Technik bekannt. Beispielsweise sind Aktivkohle und Molekularsiebe sowie metallorganische Gerüstmaterialien zu nennen.

Hierbei zeichnen sich die letztgenannten metallorganischen Gerüstmaterialien besonders dadurch aus, dass durch entsprechende Wahl des Metalls sowie des Liganden Speicher- oder Trennmaterialien erhalten werden können, die für spezielle Anwendungen eingesetzt werden können.

Dabei besteht insbesondere ein Bedarf an kostengünstigen und robusten Materialien, die sich darüber hinaus durch ein selektives Verhalten bei der Speicherung oder Trennung gegenüber bestimmten Gasen auszeichnen.

Es hat sich bei metallorganischen Gerüstmaterialien herausgestellt, dass durch geeignete Synthese Gerüstmaterialien erhalten werden können, deren Bestandteile aus konventionellen Salzen bekannt sind.

So ist beispielsweise Magnesiumformiat kommerziell erhältlich. Das Dihydrat von Magnesiumformiat mit der CAS-Nr. 6150-82-9 kann beispielsweise von der Firma Sigma Aldrich bezogen werden.

J. A. Rood et al., Inorg. Chem. 45 (2006), 5521-5528, konnten durch geeignete Synthesevorschrift ein metallorganisches Gerüstmaterial aus Magnesium und Formiat erhalten, welches porös ist, was durch Gasadsorptionsstudien unter Verwendung von Stickstoff und Wasserstoff nachgewiesen wurde.

WO 2007 118841 offenbart ein poröses Metallorganisches Gerüstmaterial aus Aluminium und Fumarsäure, welches zur Aufnahme, Speicherung oder kontrollierten Abgabe von Stoffen wie Methan verwendet werden kann.

Trotz der im Stand der Technik bekannten metallorganischen Gerüstmaterialien besteht nach wie vor ein Bedarf an metallorganischen Gerüstmaterialien, die sich insbesondere durch ein selektives Verhalten bei der Speicherung oder Trennung gegenüber bestimmten Gasen auszeichnen.

Eine Aufgabe der vorliegenden Erfindung besteht somit darin, geeignete Verwendungen zur Speicherung und Trennung sowie weitere Materialien bereitzustellen.

Die Aufgabe wird gelöst durch die Verwendung eines porösen metallorganischen Gerüstmaterials enthaltend mindestens eine erste und gegebenenfalls eine zweite organische Verbindung, wobei zumindest die erste organische Verbindung zumindest teilweise zweizähnig an mindestens ein Metallion koordinativ bindet, wobei das mindestens eine Metallion Mg (II) ist und wobei sich die erste organische Verbindung von Ameisensäure und die zweite organische Verbindung von Essigsäure ableitet, zur Speicherung oder Abtrennung von Methan.

Weiterhin wird die Aufgabe gelöst durch ein poröses metallorganisches Gerüstmaterial enthaltend mindestens eine erste und eine zweite organische Verbindung, wobei zumindest die erste organische Verbindung zumindest teilweise zweizähnig an mindestens ein Metallion koordinativ bindet, wobei das mindestens eine Metallion Mg (II) ist und wobei sich die erste organische Verbindung von Ameisensäure und die zweite organische Verbindung von Essigsäure ableitet.

Es hat sich gezeigt, dass ein poröses metallorganisches Gerüstmaterial basierend auf Magnesiumformiat zur Methanspeicherung und -trennung geeignet ist. Darüber hinaus hat sich gezeigt, dass bei der Herstellung eines Magnesiumformiat-metallorganischen Gerüstmaterials in Gegenwart von Essigsäure ein neues metallorganisches Gerüstmaterial erhalten werden kann, dessen Gerüststruktur mit dem des reinen Magnesiumformiat-Gerüstmaterials vergleichbar ist und ebenfalls zur Speicherung bzw. Abtrennung von Methan geeignet ist.

Im Rahmen der vorliegenden Erfindung ist unter dem Begriff "ableiten" zu verstehen, dass Ameisensäure und gegebenenfalls Essigsäure im porösen metallorganischen Gerüstmaterial gemäß der vorliegenden Erfindung als Formiat bzw. Acetat vorliegen, wobei auch teilweise eine protonierte Form möglich ist.

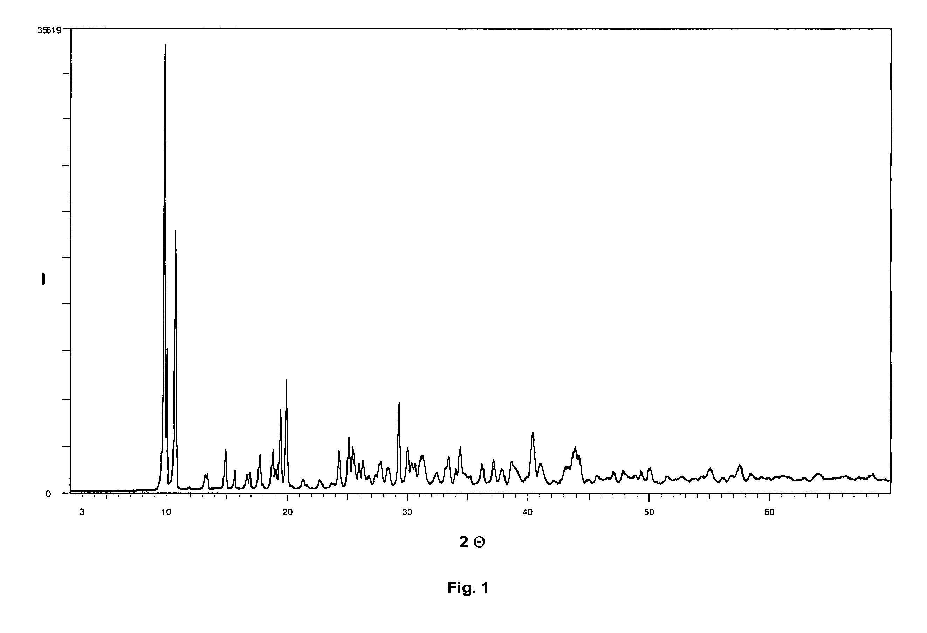

Fig. 1 zeigt das Röntendiffraktogramm des erfindungsgemäßen metallorganischen Gerüstmaterials aus Formiat und Acetat. Hierbei beschreiben in dem Diffraktogramm I die Intensität (Lᵢₙ (Counts)) und 2 Θ die 2-Theta Skala.

Vorzugsweise kennzeichnet sich das erfindungsgemäße Gerüstmaterial dadurch, dass dessen Röntgendiffraktogramm (XRD) im Bereich von 8° < 2Θ <12° zwei Reflexe aufweist, die im Bereich von 2° < 2Θ <70° die größten Reflexe darstellen.

Hierbei kann das Diffraktogramm wie folgt ermittelt werden: Die Probe wird als Pulver in den Probenbehälter eines kommerziell erhältlichen Geräts (Siemens D-5000 Diffraktometer oder Bruker D8-Advance) eingebaut. Als Strahlungsquelle wird Cu-Kα-Strahlung mit variablen Primär- und Sekundärblenden und Sekundärmonochromator benutzt. Die Detektion des Signals erfolgt über einen Szintillations- (Siemens) oder Solex-Halbleiter Detektor (Bruker). Der Messbereich für 20 wird typischerweise zwischen 2° und 70 ° gewählt. Der Winkelschritt beträgt 0,02°, die Messzeit pro Winkelschritt typischerweise 2-4s. Bei der Auswertung werden Reflexe durch eine wenigstens 3-fach höhere Signalstärke vom Grundrauschen unterschieden. Die Flächenanalyse kann manuell erfolgen, indem an die einzelnen Reflexe eine Basislinie angelegt wird. Alternativ können Programme wie zum Beispiel "Topas-Profile" der Fa. Bruker eingesetzt werden, wobei die Untergrundanpassung dann bevorzugt über ein Polynom 1. Grades in der Software automatisch erfolgt.

Weiterhin ist bevorzugt, dass das erfindungsgemäße metallorganische Gerüstmaterial neben Mg(II) keine weiteren Metallionen aufweist.

Darüber hinaus ist es ebenfalls bevorzugt, dass das erfindungsgemäße metallorganische Gerüstmaterial keine weiteren mindestens zweizähnigen organischen Verbindungen enthält, die koordinativ an das mindestens eine Metallion binden.

Vorzugsweise liegt das molare Verhältnis von erster zu zweiter organischer Verbindung im erfindungsgemäßen metallorganischen Gerüstmaterial im Bereich von 10 : 1 bis 1 : 10. Mehr bevorzugt liegt das Verhältnis im Bereich von 5 : 1 bis 1 : 5, weiter mehr bevorzugt im Bereich von 2 : 1 bis 1 : 2, weiterhin mehr bevorzugt im Bereich von 1,5 : 1 bis 1 : 1,5, weiter mehr bevorzugt im Bereich von 1,2 :1 bis 1 : 1,2, weiter mehr bevorzugt im Bereich von 1,1 : 1 bis 1 : 1,1 und insbesondere bei 1 : 1. Entsprechend können die bei der Herstellung erforderlichen Mengen an Ameisensäure und Essigsäure eingesetzt werden.

Ein weiterer Gegenstand der vorliegenden Erfindung ist ein Verfahren zur Herstellung eines erfindungsgemäßen porösen metallorganischen Gerüstmaterials, die Schritte enthaltend
(a) Umsetzen einer Reaktionslösung enthaltend Magnesiumnitrathexahydrat, Ameisensäure und Essigsäure sowie einem Lösemittel bei einer Temperatur im Bereich von 110 °C bis 150 °C für mindestens 10 Stunden und
(b) Abtrennen des ausgefallenen Feststoffes.

Das erfindungsgemäße Verfahren zur Herstellung des erfindungsgemäßen Gerüstmaterials enthält als Schritt (a) das Umsetzen einer Reaktionslösung, enthaltend Magnesiumnitrathexahydrat und Ameisensäure, Essigsäure sowie ein Lösemittel, bei einer Temperatur im Bereich von 110 °C bis 150 °C für mindestens 10 Stunden.

Bevorzugt erfolgt die Umsetzung zumindest zeitweise, insbesondere zu Beginn der Umsetzung, unter Rühren.

Als eine Ausgangsverbindung wird Magnesiumnitrathexahydrat verwendet. Vorzugsweise liegt dessen Anfangskonzentration in der Reaktionslösung im Bereich von 0,005 mol/l bis 0,5 mol/l. Weiterhin bevorzugt liegt die Anfangskonzentration im Bereich von 0,1 mol/l bis 0,4 mol/l. Insbesondere liegt die Anfangskonzentration im Bereich von 0,15 mol/l bis 0,3 mol/l.

Die Menge an Magnesiumnitrathexahydrat wird dabei in einer Menge der Reaktionslösung zugeführt, so dass aufgrund des ausgefallenen Feststoffes in Schritt (b) die Magnesiumkonzentration in der Reaktionslösung abnimmt.

Darüber hinaus ist es bevorzugt, dass das Verhältnis der anfänglichen Stoffmenge an eingesetzter Ameisensäure und Essigsäure zur anfänglichen Stoffmenge an Magnesiumnitrathexahydrat im Bereich von 2,5:1 bis 3,0:1 liegt. Weiterhin bevorzugt liegt das Verhältnis im Bereich von 2,6:1 bis 2,9:1, weiterhin bevorzugt im Bereich von 2,7:1 bis 2,8:1. Hierbei muss entsprechend die Summe der anfänglichen Stoffmengen von Ameisensäure und Essigsäure berücksichtigt werden.

Die Reaktionslösung für Schritt (a) des erfindungsgemäßen Verfahrens zur Herstellung des erfindungsgemäßen metallorganischen Gerüstmaterials enthält neben Magnesiumnitrathexahydrat und Ameisensäure sowie Essigsäure weiterhin ein Lösemittel.

Das Lösemittel muss geeignet sein, die eingesetzten Ausgangsstoffe zumindest teilweise in Lösung zu bringen. Darüber hinaus muss das Lösemittel derart gewählt werden, dass der erforderliche Temperaturbereich eingehalten werden kann.

Die Umsetzung in dem erfindungsgemäßen Verfahren zur Herstellung des erfindungsgemäßen Materials erfolgt somit in Gegenwart eines Lösemittels. Hierbei können Solvothermalbedingungen eingesetzt werden. Unter dem Begriff "thermal" ist im Rahmen der vorliegenden Erfindung ein Herstellverfahren zu verstehen, bei dem die Umsetzung in einem Druckbehälter derart durchgeführt wird, dass dieser während der Umsetzung verschlossen ist und erhöhte Temperatur angelegt wird, so dass aufgrund des Dampfdruckes von vorhandenem Lösemittel sich ein Druck innerhalb des Reaktionsmediums im Druckbehälter aufbaut. Hierdurch kann die gewünscht Umsetzungstemperatur gegebenenfalls erreicht werden.

Vorzugsweise erfolgt die Umsetzung nicht in Wasser enthaltendem Medium und ebenso nicht unter Solvothermalbedingungen.

Die Umsetzung in dem erfindungsgemäßen Verfahren erfolgt demzufolge vorzugsweise in Gegenwart eines nicht-wässrigen Lösemittels.

Die Umsetzung erfolgt vorzugsweise bei einem Druck von höchstens 2 bar (absolut). Vorzugsweise beträgt der Druck jedoch höchstens 1230 mbar (absolut). Insbesondere bevorzugt findet die Umsetzung bei Atmosphärendruck statt. Hierbei kann es jedoch apparativ bedingt zu leichten Über- oder Unterdrücken kommen. Daher ist im Rahmen der vorliegenden Erfindung unter dem Begriff "Atmosphärendruck" derjenige Druckbereich zu verstehen, der sich aus dem tatsächlichen vorliegenden Atmosphärendruck ± 150 mbar ergibt.

Die Umsetzung findet in einem Temperaturbereich von 110 °C bis 150 °C statt. Vorzugsweise liegt die Temperatur im Bereich von 115 °C bis 130 °C. Weiterhin bevorzugt liegt die Temperatur in einem Bereich von 120 °C bis 125 °C.

Die Reaktionslösung kann weiterhin eine Base aufweisen. Durch die Verwendung eines organischen Lösemittels ist es häufig nicht erforderlich, eine solche Base einzusetzen. Nichts desto trotz kann das Lösemittel für das erfindungsgemäße Verfahren derart gewählt werden, dass dieses als solches basisch reagiert, was jedoch nicht zwingend für die Durchführung des erfindungsgemäßen Verfahrens sein muss.

Ebenso kann eine Base eingesetzt werden. Bevorzugt ist jedoch, dass keine zusätzliche Base eingesetzt wird.

Es ist weiterhin vorteilhaft, dass die Umsetzung unter Rühren stattfinden kann, was auch bei einem Scale-up vorteilhaft ist.

Das (nicht-wässrige) organische Lösemittel ist vorzugsweise ein C₁₋₆-Alkanol, Dimethylsulfoxid (DMSO), N,N-Dimethylformamid (DMF), N,N-Diethylformamid (DEF), N,N-Dimethylacetamid (DMAc), Acetonitril, Toluol, Dioxan, Benzol, Chlorbenzol, Methylethylketon (MEK), Pyridin, Tetrahydrofuran (THF), Essigsäureethylester, gegebenenfalls halogeniertes C₁₋₂₀₀-Alkan, Sulfolan, Glykol, N-Methylpyrrolidon (NMP), gamma-Butyrolacton, alicyclische Alkohole wie Cyclohexanol, Ketone, wie Aceton oder Acetylaceton, Cycloketone, wie Cyclohexanon, Sulfolen oder Mischungen davon.

Ein C₁₋₆-Alkanol bezeichnet einen Alkohol mit 1 bis 6 C-Atomen. Beispiele hierfür sind Methanol, Ethanol, n-Propanol, i-Propanol, n-Butanol, i-Butanol, t-Butanol, Pentanol, Hexanol sowie Gemische davon.

Ein gegebenenfalls halogeniertes C₁₋₂₀₀-Alkan bezeichnet ein Alkan mit 1 bis 200 C-Atomen, wobei ein oder mehrere bis hin zu allen Wasserstoffatomen durch Halogen, vorzugsweise Chlor oder Fluor, insbesondere Chlor, ersetzt sein kann bzw. können. Beispiele hierfür sind Chloroform, Dichlormethan, Tetrachlormethan, Dichlorethan, Hexan, Heptan, Oktan sowie Gemische davon.

Bevorzugte Lösemittel sind DMF, DEF, DMAc und NMP. Besonders bevorzugt ist DMF.

Der Begriff "nicht-wässrig" bezieht sich vorzugsweise auf ein Lösemittel, das einen Höchstwassergehalt von 10 Gew.-%, mehr bevorzugt 5 Gew.-%, weiterhin mehr bevorzugt 1 Gew.-%, weiterhin bevorzugt 0,1 Gew.-%, besonders bevorzugt 0,01 Gew.-% bezogen auf das Gesamtgewicht des Lösemittels nicht überschreitet.

Vorzugsweise beträgt der Höchstwassergehalt während der Umsetzung 10 Gew.-%, mehr bevorzugt 5 Gew.-% und weiterhin mehr bevorzugt 1 Gew.-%.

Der Begriff "Lösemittel" betrifft reine Lösemittel sowie Gemische von unterschiedlichen Lösemitteln.

Schritt (a) des erfindungsgemäßen Verfahrens zur Herstellung des erfindungsgemäßen Gerüstmaterials wird für mindestens 10 Stunden durchgeführt. Vorzugsweise erfolgt die Umsetzung mindestens einen Tag, weiter bevorzugt mindestens zwei Tage.

Weiterhin weist das erfindungsgemäße Verfahren den Schritt (b), Abtrennen des ausgefallenen Feststoffes, auf.

Aufgrund von Schritt (a) des erfindungsgemäßen Herstellverfahrens fällt das Gerüstmaterial als Feststoff aus der Reaktionslösung aus. Eine Abtrennung erfolgt durch im Stand der Technik bekannte Methoden, wie Filtration oder dergleichen.

Das rein auf Magnesiumformiat basierte poröse metallorganische Gerüstmaterial kann gemäß dem oben durchgeführten Verfahren oder gemäß der Synthese, wie sie in J. A. Rood et al., Inorg. Chem. 45 (2006), 5521-5528, beschrieben ist, erhalten werden.

Sowohl das rein Magnesiumformiat-basierte metallorganische Gerüstmaterial als auch das sowohl Formiat als auch Acetat enthaltende Magnesium-basierte poröse metallorganische Gerüstmaterial können zur Speicherung und Abtrennung von Methan eingesetzt werden. Hierbei ist die Verwendung des Gerüstmaterials, sowohl Acetat als auch Formiat enthaltend, bevorzugt.

Dementsprechend besteht eine bevorzugte Ausführungsform in der Verwendung eines porösen metallorganischen Gerüstmaterials zur Speicherung oder Abtrennung von Methan, wobei das Gerüstmaterial die erste und die zweite organische Verbindung enthält.

Eine bevorzugte Verwendung liegt darin, dass Methan aus einem Gasgemisch mit Hilfe eines erfindungsgemäßen metallorganischen Gerüstmaterials abgetrennt wird.

Das Gasgemisch weist hierbei vorzugsweise neben Methan Kohlenmonoxid und/oder Wasserstoff auf.

Ein weiterer Gegenstand der vorliegenden Erfindung ist somit die bevorzugte Verwendung des erfindungsgemäßen metallorganischen Gerüstmaterials sowie des rein Magnesiumformiat-basierten Gerüstmaterials zur Abtrennung von Methan aus einem Gasgemisch, wobei das Gasgemisch neben Methan mindestens eines der Gase, ausgewählt aus der Gruppe bestehend aus Kohlenmonoxid und Wasserstoff, aufweist.

Aufgrund der aufgeführten Verwendung des erfindungsgemäßen Gerüstmaterials sowie des Reinmagnesiumformiat-basierten Gerüstmaterials besteht ein weiterer Gegenstand der vorliegenden Erfindung in einem Verfahren zur Speicherung oder Abtrennung von Methan, den Schritt enthaltend
- Inkontaktbringen des Methans oder eines Methan enthaltenden Gasgemisches mit einem entsprechenden metallorganischen Gerüstmaterial.

Die Gasadsorption bzw. -trennung erfolgt grundsätzlich nach dem Stand der Technik bekannten Verfahren.

Grundlagen und technische Verfahren sind beispielsweise in Werner Kast, Adsorption aus der Gasphase, VCH Weinheim, 1988, beschrieben.

Die Druckwechseladsorption ist beispielsweise in D. M. Ruthwen et al., Wiley-VCH, 1993, beschrieben.

### Beispiele

Beispiel 1 Herstellung eines Magnesiumformiat-Acetat enthaltenden metallorganischen Gerüstmaterials

### Ansatz:

| | | | |
|---|---|---|---|
| 1) | Magnesiumnitrat*6 H₂O | 38,5 mmol | 9,90g |
| 2) | Ameisensäure | 53,2 mmol | 2,5g |
| 3) | Essigsäure | 53,2 mmol | 3,2g |
| 4) | N,N-Dimethylformamid (DMF) | 2,19 mol | 160,0g |

Es wird das Magnesiumnitrat in DMF in einem Autoklavenbecher aufgelöst. Dazu gibt man eine Lösung aus der Ameisensäure und Essigsäure und rührt die Lösung für 10 min.

### Kristallisation:

### 125°C/78h

### Ausbau:

Klare Lösung mit weißen Kristallen. Die Lösung hat eine pH von 6,67

### Aufarbeitung:

Die Kristalle werden abfiltriert, 2-mal mit 50 ml DMF gewaschen.
Auswaage: 4,763g

### Feststoffgehalt:

Auswaage: 2,7% Feststoff

Fig. 1 zeigt das XRD des erhaltenen Materials, wobei I die Intensität (Lᵢₙ (Counts)) und 2 Θ die 2-Theta Skala angibt.

Beispiel 2 Herstellung eines Magnesiumformiat-basierten metallorganischen Gerüstmaterials

| | | | |
|---|---|---|---|
| 1) | Magnesiumnitrat*6Wasser | 38,5 mmol | 9,90g |
| 2) | Ameisensäure | 106,5 mmol | 4,8g |
| 3) | DMF | 2,19 mol | 160,0g |

Es wird das Magnesiumnitrat in DMF in einem Autoklavenbecher aufgelöst. Dazu gibt man die Ameisensäure und rührt die Lösung für 10 min. (pH=3,49)

### Kristallisation:

### 125 °C / 78h

### Ausbau:

Klare Lösung mit weißen Kristallen

### Aufarbeitung:

Die Kristalle werden abfiltriert, 2-mal mit 50 ml DMF gewaschen.
Auswaage: 5,162g

### Feststoffgehalt:

Auswaage: 2,9% Feststoff

### Beispiel 3 Adsorptionsmessungen

Für das Gerüstmaterial aus Beispiel 1 und das Magnesiumformiat-basierte Gerüstmaterial gemäß Beispiel 2 werden Adsorptionsmessungen durchgeführt.

Fig. 2 zeigt die Aufnahme von Methan (oberer Graph) bei 298 K sowie Kohlenmonoxid (mittlerer Graph) und Wasserstoff (unterer Graph) bei 313 K für das Gerüstmaterial aus Beispiel 1.

Fig. 3 zeigt ebenfalls die Aufnahme von Methan (oberer Graph), Kohlenmonoxid (mittlerer Graph) und Wasserstoff (unterer Graph) mit Hilfe des im Stand der Technik bekannten Magnesiumformiat-basierten metallorganischen Gerüstmaterials unter den gleichen Bedingungen, wie diejenigen, welche bei den Messungen für Fig. 2 eingehalten wurden.

Wie sich aus den Reinstoffisothermen ergibt, ist eine Speicherung von Methan sowie die Trennung von Methan aus einem Methan-haltigen Gemisch, das darüber hinaus Kohlenmonoxid und Wasserstoff enthält, möglich.

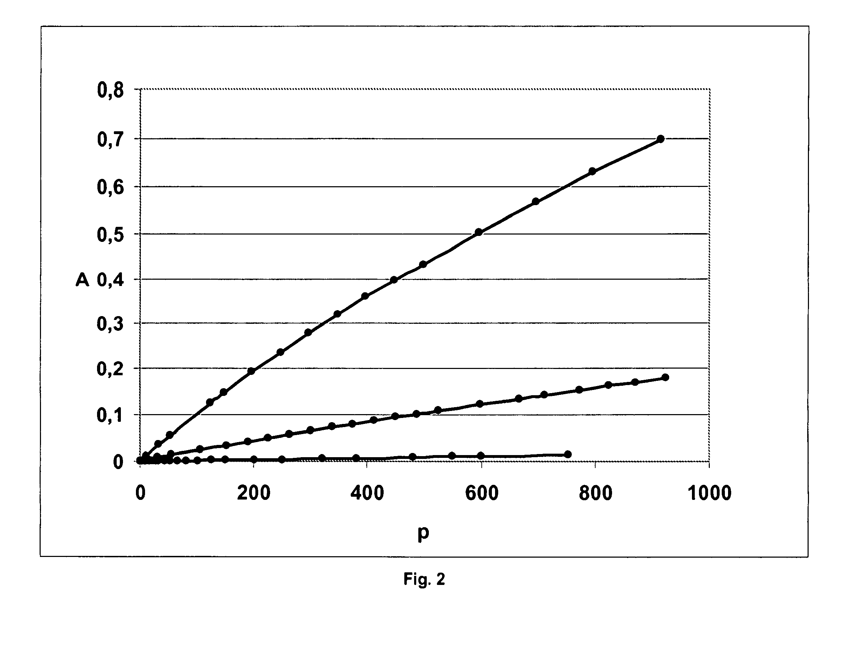

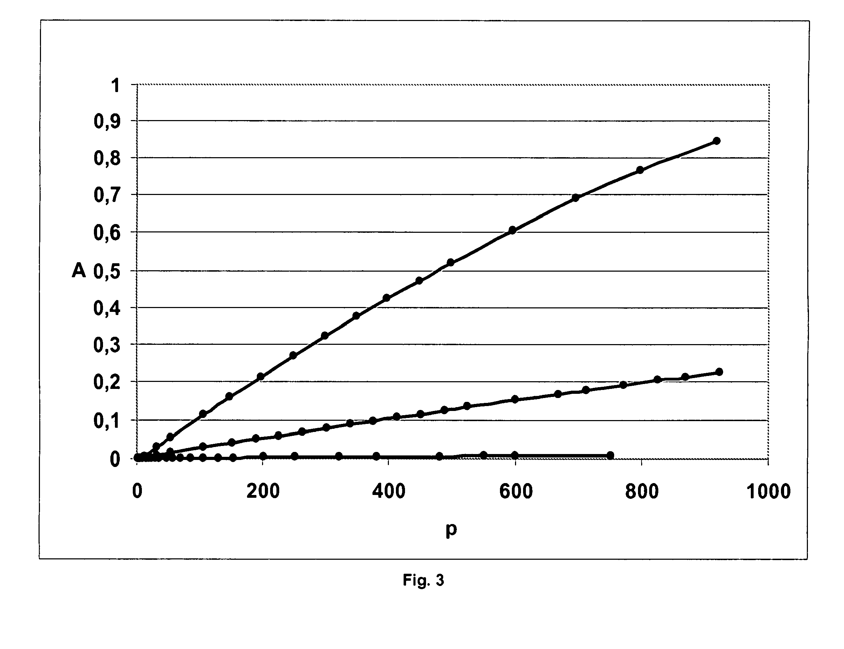

In den Fig. 2 und 3 ist die Aufnahme A (in mmol/g) als Funktion des absoluten Drucks p (in mmHg) dargestellt.

## Patentansprüche

1. Verwendung eines porösen metallorganischen Gerüstmaterials enthaltend mindestens eine erste und gegebenenfalls eine zweite organische Verbindung, wobei zumindest die erste organische Verbindung zumindest teilweise zweizähnig an mindestens ein Metallion koordinativ bindet, wobei das mindestens eine Metallion Mg (II) ist und wobei sich die erste organische Verbindung von Ameisensäure und die zweite organische Verbindung von Essigsäure ableitet, zur Speicherung oder Abtrennung von Methan.

2. Verwendung nach Anspruch 1, **dadurch gekennzeichnet, dass** das Gerüstmaterial die erste und die zweite organische Verbindung enthält.

3. Verwendung nach Anspruch 1 oder 2, **dadurch gekennzeichnet, dass** Methan aus einem Gasgemisch abgetrennt wird, wobei das Gasgemisch neben Methan mindestens eines der Gase ausgewählt aus der Gruppe bestehend aus Kohlenmonoxid und Wasserstoff aufweist.

4. Verfahren zur Speicherung oder Abtrennung von Methan den Schritt enthaltend
• In Kontakt bringen des Methans oder eines Methan enthaltenen Gasgemisches mit einem metallorganischen Gerüstmaterial, wie in Anspruch 1 oder 2 angegeben.

5. Poröses metallorganisches Gerüstmaterial enthaltend mindestens eine erste und eine zweite organische Verbindung, wobei zumindest die erste organische Verbindung zumindest teilweise zweizähnig an mindestens ein Metallion koordinativ bindet, wobei das mindestens eine Metallion Mg (II) ist und wobei sich die erste organische Verbindung von Ameisensäure und die zweite organische Verbindung von Essigsäure ableitet.

6. Gerüstmaterial nach Anspruch 5, **dadurch gekennzeichnet, dass** dessen Röntgendiffraktrogramm (XRD) im Bereich von 8° < 2Θ < 12° zwei Reflexe aufweist, die im Bereich von 2° < 2Θ < 70° die größten Reflexe darstellen.

7. Gerüstmaterial nach Anspruch 5 oder 6, **dadurch gekennzeichnet, dass** das metallorganische Gerüstmaterial neben Mg (II) keine weiteren Metallionen aufweist.

8. Gerüstmaterial nach einem der Ansprüche 5 bis 7, **dadurch gekennzeichnet, dass** das metallorganische Gerüstmaterial keine weiteren mindestens zweizähnigen organischen Verbindungen enthält, die koordinativ an das mindestens eine Metallion binden.

9. Gerüstmaterial nach einem der Ansprüche 5 bis 8, **dadurch gekennzeichnet, dass** das molare Verhältnis von erster zu zweiter organischer Verbindung im Bereich von 10 : 1 bis 1 : 10 liegt.

10. Verfahren zur Herstellung eines porösen metallorganischen Gerüstmaterials nach einem der Ansprüche 5 bis 9, die Schritte enthaltend
(a) Umsetzen einer Reaktionslösung enthaltend Magnesiumnitrathexahydrat, Ameisensäure und Essigsäure sowie einem Lösemittel bei einer Temperatur im Bereich von 110 °C bis 150 °C für mindestens 10 Stunden und
(b) Abtrennen des ausgefallenen Feststoffes.

## Claims

1. The use of a porous metal organic framework comprising at least a first organic compound and optionally a second organic compound, in which at least the first organic compound is at least partially coordinated in a bidentate fashion to at least one metal ion, where the at least one metal ion is Mg(II) and the first organic compound is derived from formic acid and the second organic compound is derived from acetic acid, for storing or separating off methane.

2. The use according to claim 1, wherein the framework comprises the first organic compound and the second organic compound.

3. The use according to claim 1 or 2, wherein methane is separated off from a gas mixture comprising methane together with at least one gas selected from the group consisting of carbon monoxide and hydrogen.

4. A method of storing or separating off methane, which comprises the step of
• contacting of the methane or a methane-comprising gas mixture with a metal organic framework as defined in claim 1 or 2.

5. A porous metal organic framework comprising at least a first organic compound and a second organic compound, in which at least the first organic compound is at least partially coordinated in a bidentate fashion to at least one metal ion, where the at least one metal ion is Mg(II) and the first organic compound is derived from formic acid and the second organic compound is derived from acetic acid.

6. The framework according to claim 5 whose X-ray diffraction pattern (XRD) displays two reflections in the range 8° **< 2Θ < 12° which are the strongest reflections in the range 2° < 2Θ < 70°.**

7. The framework according to claim 5 or 6 which comprises no further metal ions in addition to Mg(II).

8. The framework according to any of claims 5 to 7 which comprises no further at least bidentate organic compounds which can coordinate to the at least one metal ion.

9. The framework according to any of claims 5 to 8, wherein the molar ratio of the first organic compound to the second organic compound is in the range from 10 : 1 to 1 : 10.

10. A process for preparing a porous metal organic framework according to any of claims 5 to 9, which comprises the steps
(a) reaction of a reaction solution comprising magnesium nitrate hexahydrate, formic acid and acetic acid and also a solvent at a temperature in the range from 110°C to 150°C for at least 10 hours and
(b) isolation of the precipitated solid.

## Revendications

1. Utilisation d'un matériau structurel métallo-organique poreux contenant au moins un premier et le cas échéant un deuxième composé organique, où au moins le premier composé organique se lie au moins partiellement par coordination de manière bidentate à au moins un ion métallique, ledit au moins un ion métallique étant Mg (II) et le premier composé organique étant dérivé d'acide formique et le deuxième composé organique étant dérivé d'acide acétique, pour l'accumulation ou la séparation de méthane.

2. Utilisation selon la revendication 1, **caractérisée en ce que** le matériau structurel contient le premier et le deuxième composé organique.

3. Utilisation selon la revendication 1 ou 2, **caractérisée en ce que** le méthane est séparé à partir d'un mélange gazeux, le mélange gazeux présentant, outre le méthane, au moins un des gaz choisis dans le groupe constitué par le monoxyde de carbone et l'hydrogène.

4. Procédé pour l'accumulation ou la séparation de méthane, contenant l'étape
- de mise en contact du méthane ou d'un mélange gazeux contenant du méthane avec un matériau structurel métallo-organique, tel qu'indiqué dans la revendication 1 ou 2.

5. Matériau structurel métallo-organique poreux contenant au moins un premier et un deuxième composé organique, où au moins le premier composé organique se lie au moins partiellement par coordination de manière bidentate à au moins un ion métallique, ledit au moins un ion métallique étant Mg (II) et le premier composé organique étant dérivé d'acide formique et le deuxième composé organique étant dérivé d'acide acétique.

6. Matériau structurel selon la revendication 5, **caractérisé en ce que** son diffractogramme aux rayons X (XRD) dans la plage de 8° < 2θ < 12° présente deux réflexions qui représentent, dans la plage de 2° < 2θ < 70°, les plus grandes réflexions.

7. Matériau structurel selon la revendication 5 ou 6, **caractérisé en ce que** le matériau structurel métallo-organique ne présente pas d'autres ions métalliques à côté du Mg (II).

8. Matériau structurel selon l'une quelconque des revendications 5 à 7, **caractérisé en ce que** le matériau structurel métallo-organique ne contient pas d'autres composés organiques au moins bidentates qui se lient par coordination audit au moins un ion métallique.

9. Matériau structurel selon l'une quelconque des revendications 5 à 8, **caractérisé en ce que** le rapport molaire du premier au deuxième composé organique se situe dans la plage de 10:1 à 1:10.

10. Procédé pour la préparation d'un matériau structurel métallo-organique poreux selon l'une quelconque des revendications 5 à 9 comprenant les étapes
(a) de transformation d'une solution réactionnelle contenant du nitrate de magnésium hexahydraté, de l'acide formique et de l'acide acétique ainsi qu'un solvant à une température dans la plage de 110°C à 150°C pendant au moins 10 heures et
(b) de séparation du solide précipité.
